# EUROPEAN PATENT SPECIFICATION

(11) **EP 1 465 566 B1**
(45) Date of publication and mention of the grant of the patent: **25.09.2013**
(21) Application number: 02793624.4
(22) Date of filing: 11.12.2002
(51) Int. Cl.: A61F 13/491, A61F 13/496, A61F 13/49

(54) **ABSORBENT ARTICLE**
ABSORBIERENDER ARTIKEL
ARTICLE ABSORBANT

(30) Priority: 28.12.2001 SE 0104436
(43) Date of publication of application: 13.10.2004
(73) Proprietor: SCA Hygiene Products AB, 405 03 Göteborg (SE)
(72) Inventor: SANDIN, Cécile, S-431 36 Mölndal (SE); E N, Hans, S-435 41 Mölnlycke (SE); HERMANSSON, Kent, S-426 54 Västra Frölunda (SE); STRANNEMALM, Kenneth, S-448 31 Floda (SE); ELFSTRÖM, Anna-Carin, S-423 38 Torslanda (SE); LINDMARK, Karin, S - 425 42 Hisings Kärra (SE)
(74) Representative: Olsson, Stefan
(86) International application number: PCT/SE2002/002288
(87) International publication number: WO 2003/059224

(56) References cited:
- EP-A1- 0 560 630
- WO-A1-01/13851
- WO-A1-01/15898
- SE-A- 9 904 202
- US-A1- 2001 000 529

## Description

### TECHNICAL FIELD

The present invention relates to an absorbent article in form of non-refastenable absorbent pants, such as incontinence pants, nappy pants or sanitary towel pants, said absorbent article has a longitudinal direction and a transverse direction and comprising an elastic waist portion, an absorbent element, which has a length and a width and is intended to at least cover the genitals of the wearer during use of the article, and a liquid-impermeable outer layer which is intended to enclose the absorbent element on at least that side thereof which faces away from the wearer during use of the article. The invention also relates to a method of manufacturing such absorbent articles of pants-like shape.

### BACKGROUND ART

Absorbent disposable products for taking up urine, faeces or menstrual blood have developed greatly since they came into more general use during the 1960s and 1970s. As they are disposable products, it is necessary that they can be manufactured and sold at a very low price. At the same time, it is important that the products function well and reliably. Good fit and comfort are also important characteristics. The first disposable nappies were made of two-part products, consisting of an outer pant made of plastic, intended to be re-used, and a rectangular absorbent insert for disposable use. The absorbent material in these inserts initially consisted of cellulose tissue. Later, better absorption materials made of what is known as fluff pulp made of cellulose were developed. The fit and comfort of these early nappies were poor. The products were unwieldy and uncomfortable for the wearer. Towards the end of the 1970s, the first complete disposable nappies arrived, that is to say nappies in which the absorption bodies were integrated with a liquid-impermeable outer layer. The absorption materials have developed and improved, which has resulted in the possibility of the absorption bodies being adapted better to the anatomy of the wearer. Hourglass-shaped absorption bodies with a narrower crotch portion between the two end portions are now predominant. The trend has also been towards increasingly thin products, which has been made possible by the inclusion of what are known as superabsorbent materials in the absorbent element. There are many reasons why thinner and even smaller absorption bodies are desirable. A thinner, smaller absorption body is more comfortable and more discreet, which is especially important for adult incontinent wearers. A reduction in volume is also very important financially because the product then requires less storage space and is easier to transport and takes up less shelf space in shops. This is important for the financial management of the shops, and if a manufacturer can produce products requiring less space in the shops than the products of the competitors, this affords a not inconsiderable competitive advantage. Moreover, there is increased pressure from authorities, in particular as far as disposable articles are concerned, to use as little material as possible for the purpose of reducing the burden on the environment

The smaller the absorption bodies become, the more important it becomes that the absorption bodies come to lie in the correct place directly in front of the genitals of the wearer and remain in place during use even when the wearer is very active and moves a great deal. The demands of consumers for discretion, comfort and reliable functioning are also increasingly exacting. Requirements for the absorption element to come to lie correctly when put on and then be retained in the correct place have therefore increased the need for good fixing of the article on the body and the need for very good adaptability to the body when the wearer moves, at the same time as requirements have increased for the article always to come to lie in the correct place when the article is put on the wearer. This has led to the development of what are known as nappy pants, which have elastic portions for improved fit and comfort and increased flexibility during movements of the wearer compared with conventional absorbent articles.

An early patent publication relating to nappy pants of the disposable type is GB 2 112 267-A. However, this publication from 1983 discloses primitive nappy pants which did not become a commercial product. Not until the 1990s did absorbent articles of pants-like shape and construction become a mayor commercial product. Pants-like articles now exist in the form of nappies for infants and nappies for adults and to some extent sanitary towel pants for absorption of menstrual fluid. Previously commercially available nappy pants have been designed in principle like conventional nappies with a front portion and a rear portion and also an intermediate crotch portion, the front and rear portions being interconnected by a side seam between each leg opening and the waist opening of the pants. The nappy pants have been produced by plane nappy-like pieces being produced in a continuous web, the individual nappy pants being formed by nappy-like blanks being folded double and provided with said side seams to form nappy pants. These side seams project laterally from the finished product and are undesirable because they protect and interfere with the fit of garments worn over the top. On account of their shape, they are visible through garments wom on top of the nappy pants and fitting closely around the body. They can also snag in clothing and even cause tears in nylon tights. Such projecting side seams can also chafe and give rise to pressure sores on wearers who spend a lot of time lying on their side.

WO 00/61049 has proposed improved nappy pants, in which the projecting sides seams have been eliminated. In this construction, the side seams have been eliminated by virtue of elastic side portions extending continuously in one piece from the front portion of the nappy pants to their rear portion on both sides of the nappy pants. However, the nappy pants according to WO 00/61049 have a number of disadvantages. The nappy pants according to said publication have what is referred to as a chassis, which extends over the entire nappy pants and forms the front portion, the crotch portion and the rear portion and is most reminiscent of a conventional nappy, and also said elastic side panels which each overlap the chassis at both the front and the rear on the nappy pants. These overlapping portions do not serve any actual purpose on the finished product and are in fact undesirable because a lot of material is wasted, that is to say they are used for no other purpose than joining together. The overlapping, joined-together portions are less of a nuisance and less uncomfortable for the wearer than the projecting side seams on previously known nappy pants. Owing to their unfavourable positioning, particularly at the rear, the overlapping portions can still give rise to a risk of chafing and back sores on wearers who spend a lot of time lying on their back. Another disadvantage of the nappy pants according to WO 00/61049 is that said chassis is relatively large and, as this portion is relatively rigid at least in comparison with the elastic side portions of the nappy pants, the nappy pants as a whole are not as adaptable to the body of the wearer but there is a risk that the nappy pants will be displaced from their optimum position in relation to the body when a wearer lies in bed and moves. In particular the rigid rear portion of the nappy pants can be displaced and pull both the crotch portion-and the front portion from their optimum positions directly in front of the genitals of the wearer because the front and rear portion are essentially rigidly joined together in one piece with the crotch portion.

An essential problem with absorbent articles of pants-like shape is that conventional articles of this kind tightly enclose the area around the genitals of the wearer, whereby this area, when body fluids are excreted, becomes locally saturated with moisture and vapour, which has an adverse effect on the skin of the wearer. In some cases disturbing skin irritation can occur. Furthermore, also minor chafing of the article against the skin of the wearer, in combination with a moist environment and during lengthy usage of the article, can give rise to sores on older people with flesh that heals poorly.

Breathable and vapour-permeable but liquid-impermeable materials for use in absorbent articles, such as nappies, incontinence products or sanitary towels, are previously known. Breathable materials can consist of perforated plastic films, as described in US 5,628,737, or of micro-porous plastic films, as described in, for example, EP-A-0238200. Another example of breathable materials are so-called non-woven materials which are air-permeable and vapour-permeable and which, if required, have been treated with means to improve the liquid-impermeability, as is described in EP-A-0196654. In absorbent articles of pants-like shape, with a fit which tightly encloses around the wearer, the need for air-permeability and vapour-permeability is especially important. This in combination with all the above-mentioned problems associated with absorbent articles of pants-like shape has resulted in a great need to find a pants-like article which functions well in all respects and which, in addition, it is possible to manufacture rationally.

### DISCLOSURE OF INVENTION

By means of the present invention, an improved absorbent article of pants-like shape has been produced.

An article of the type referred to in the introduction is to this end characterized in that said elastic waist portion is made from an elastic first piece which has a length and a width and which is elongate in a length direction parallel to said transverse direction and is in use intended to partly surround the trunk of the wearer and to form a rear portion and side portions of the pants-like article, in that a second piece forming part of the article is designed to form a front portion and a crotch portion of the pants-like article, in that said second piece has a length and a width and is elongate along the longitudinal direction with two opposite end edges and two opposite longitudinal side edges, in that the width of the second piece is, at least in the crotch portion, smaller than the length of the first piece, in that the second piece is arranged with its length direction at right angles in relation to the length direction of the first piece and is connected by a first end portion to one edge portion along the length direction of the first piece, centrally thereon, in that
one end portion of the first piece is connected to a first side edge portion of one of said side edges of the second piece, and in that the other end portion of the first piece is connected in a corresponding manner to a second side edge portion of the other of said side eges of the second piece, in addition to which the absorbent element is in its entirety arranged on the second piece, and that the liquid-impermeable outer layer is breathable.

By virtue of the fact that the entire rear portion as well is elastic and, together with the elastic side portions, forms a single continuous elastic first piece, the pants as a whole are more adaptable to body movements. Local irregularities are taken up and smoothed out by the continuous elastic piece and are not transferred to the more rigid parts of the front portion and a crotch portion located directly in front of the genitals of the wearer. Compared with conventional nappies and previously known absorbent articles of pants-like shape, the article according to the present invention affords superior fit and comfort. Most of the pants-like garment is completely smooth. The seams required are arranged at the transition between the front portion and the first piece and at the transition between the first and the second piece in the crotch portion. These seams come to lie in places which are not subjected to any significant pressure during use of the article, and there is less risk of chafing and pressure sores caused by seams on the pants-like article. The risk of chafing, pressure sores or other skin irritation diminishes also due to the fact that the article is air-permeable and vapour-permeable in front of the absorbent element where large quantities of liquid are absorbed locally.

The design of the absorbent article according to the invention in only two part pieces, where the absorbent element is in its entirety located on the second part piece, affords greater freedom of choice with regard to manufacturing method compared with previously known articles of pants-like shape.

According to one embodiment, the invention is characterized in that the length of the second piece is greater than the width of the first piece, and in that the projecting portion of the second piece formed by the length difference is in its entirety located below that side edge of the first piece which is the lower one during use of the article, and there forms the crotch portion. According to another embodiment, the invention is in this connection characterized in that said projecting portion has a smaller width than the remainder of the second piece.

According to another embodiment, the invention is characterized in that the absorbent element extends in its longitudinal direction over the entire crotch portion and a little way up over the front portion in the direction of that side edge of the first piece which is the upper one during use of the article.

According to another embodiment, the invention is characterized in that the absorbent element is arranged so as to extend with its lower end portion only a little way over the crotch portion and over less than half the extent of said projecting portion. According to another embodiment, the invention is in this connection characterized in that the absorbent element tapers in the direction of the crotch portion.

According to an embodiment in which the absorbent element extends over the crotch portion, the invention is characterized in that the absorbent element forms a concave shape with its side edge portions in the crotch area.

According to one embodiment, the invention is characterized in that said connected side edge portions and end portions of said first and second pieces are arranged in an overlapping manner, with the inside of an overlapping portion being arranged against the outside of an overlapped portion.

According to another embodiment, the invention is characterized in that the second piece includes a liquid-permeable inner layer and said liquid-impermeable outer layer, and in that the absorbent element is arranged between said inner and outer layers, the inner and outer layers extending in the lateral direction and longitudinal direction outside the absorbent element and being interconnected there.

According to one embodiment, the invention is characterized in that the liquid-impermeable and breathable layer only covers the absorbent element. According to one particular embodiment, the invention is thereby distinguished in that the absorbent element is made up of fibres and that the liquid-impermeable and breathable outer layer consists of an outer layer of hydrophobic fibres connected with the absorbent element.

According to one embodiment, the inventon is characterized in that said breathable portion has an MTVR-value between in the order of magnitude of 200-10000 g/m2/24h. The term MTVR stands for "Moisture Vapour Transmission Rate", i.e. the amount of vapour which can pass per unit area during a certain period of time. Said breathable portion suitably has an MTVR-value in the order of magnitude of 500-5000 g/m2/24h and most preferably in the order of magnitude of 1000-3000 g/m2/24h.

Measurement of breathability has been performed with-the method ASTM E96 and the measurement equipment Lyssy has been used.

According to one embodiment, the entire second piece is breathable.

According to a further embodiment, the first piece is rectangular, which simplifies the manufacturing.

According to one embodiment, the second piece has a concave portion between the first piece and up to said side edge portions of the second piece, wherein the concave portion forms cut-outs for the legs of the wearer. According to another embodiment, the second piece is, at least along a part of its length, narrowing towards the first piece, wherein the narrowing portion forms cut-outs for the legs of the wearer.

According to one embodiment, the invention is characterized in that the second piece is essentially inelastic. The term Inelastic here denotes that the front section, under normally occurring stresses, does not stretch.

According to another embodiment, the invention is characterized in that the elastic members are arranged along the edges of the second piece, along at least the crotch portion for the forming of leg elastics, intended to lie in sealing contact with the legs of the wearer. According to one embodiment, the invention is further characterized in that the elastic members extend from an area an the rear portion where the elastic first piece and the second piece overlap each other and further along the crotch portion along the edges of the second piece in a direction towards the front portion, whereby the elastic members are effectively connected with the elastic first piece.

In such a design, the leg elastic is connected with the waist elastic in the first piece, which gives the pants-like article a good fit.

According to a further embodiment, the invention is characterized in that the absorbent element comprises superabsorbent material and is continuously narrowing in a direction towards the rear portion of the article, and that the concentration of superabsorbent material increases in the same direction.

According to a suitable embodiment, the invention is characterized in that the liquid-impermeable outer layer is breathable and vapour-permeable, and that the rest of the article, over its entire area, is breathable and vapour-permeable.

According to a modified embodiment, the invention is characterized in that different portions of the article due to the choice of different materials or degree of perforation have different degrees of breathability.

According to one embodiment, the invention is characterized in that the elastic first piece has a higher degree of breathability than the liquid-impermeable outer layer on the second piece.

A method of manufacturing an article of the type mentioned in the introduction is characterized according to the invention in that said elastic waist portion is made from an elastic first piece which is essentially rectangular with a length and a width and is intended in use to partly surround the trunk of the wearer and to form a rear portion and side portions of the pants-like article, in that a second piece forming part of the article is designed to form a front portion and a crotch portion of the pants-like article, in that said second piece is made with a length and a width and is arranged with its length direction along the longitudinal direction with two opposite end edges and two opposite longitudinal side edges, in that the width of the second piece is designed, at least in the crotch portion, smaller than the length of the first piece, in that the second piece is arranged with its length direction at right angles in relation to the length direction of the first piece and is connected by a first end portion to one edge portion along the length direction of the first piece, centrally thereon, in that one end portion of the first piece is connected to a first side edge portion of one of said side edges of the second piece, and in that the other end portion of the first piece is connected in a corresponding manner to a second side edge portion of the other of said side edges of the second piece, and that the liquid-impermeable outer layer, which is intended to cover the absorbent element is formed from a breathable material. According to one embodiment, the method is in this connection characterized in that the absorbent element is in its entirety arranged on the second piece forming part of the article before said piece is connected to said first piece. According to a further embodiment, the method according to the invention is characterized in that the absorbent element, before being arranged on the second piece, is provided with a liquid-impermeable and breathable layer.

### DESCRIPTION OF DRAWINGS

The invention will be described in greater detail below with reference to illustrative embodiments shown in the accompanying drawings, in which
- Fig. 1: shows schematically a phase of the construction of an absorbent article according to a first embodiment.
- Fig. 2: shows schematically in plane form an assembled article according to the first embodiment.
- Fig. 3: shows the article according to Fig. 2 in perspective.
- Fig. 4: shows a section along the line IV-IV in Fig. 1.
- Fig. 5: shows schematically a phase of the construction of an absorbent article according to a second embodiment.
- Fig. 6: shows in plane form an assembled article according to the second embodiment.
- Fig. 7: shows in plane form an assembled article according to a third embodiment.
- Fig. 8: shows in plane view an assembled article according to a fourth embodiment of the invention.
- Fig. 9: shows schematically the distribution of superabsorbent material in one embodiment of an absorbent element belonging to the article.
- Fig. 10: shows a section along the line X-X in Fig. 8.

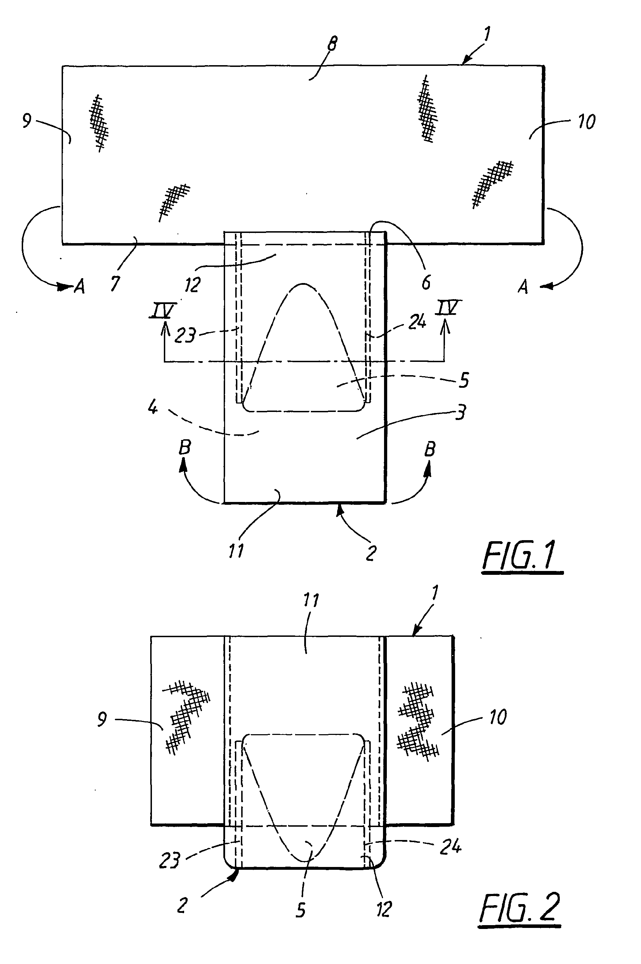

As can be seen from Fig. 1, the article in the embodiment according to Fig. 1 comprises a first piece 1. This piece is elastically stretchable and is shown in Fig. 1 in a plane and even extended state, in which the elastic piece 1 is essentially rectangular. The elastic first piece 1 can be made from conventional materials well-known to the person skilled in the art, such as woven elastic materials, elastic non-wovens, elastic laminates or elastic films. Another alternative is a material with prestressed elastic threads arranged on a supporting material, for example non-woven. The important factor is that the elastic piece is stretchable for a wearer when the article is put on, and that it fits closely with suitable tightness around the wearer during use of the article. The tightness is of course regulated by means of size and elastic stretchability.

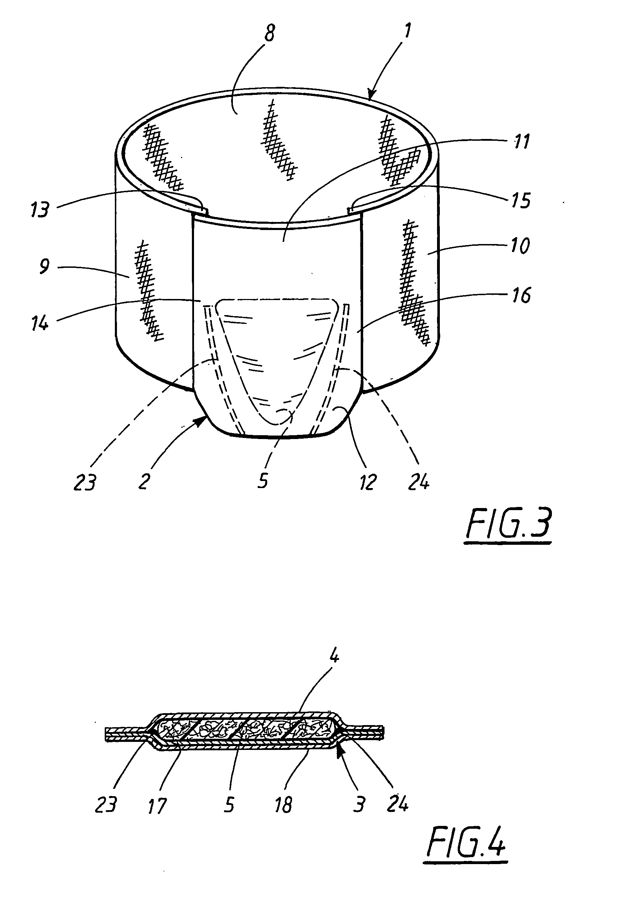

The article includes a second piece 2 which, as can be seen from Figs. 1 and 4, consists of an outer layer 3, an inner layer 4 and an absorbent element 5 arranged between these. In the embodiment in plane form shown in Fig. 1, this element is essentially triangular. The material selected for the absorption element is not critical, but it can be chosen from among materials or material combinations well-known to the person skilled in the art. For example, the absorbent element can consist of cellulose fluff pulp with superabsorbent materials in powder or fibre form added in. The absorbent element can also consist of a foam material with or without superabsorbent material.

The outer layer 3 must be liquid-impermeable and breathable. The breathable and liquid-impermeable layer 3 must have a so-called MTVR-value in the order of magnitude of 200-10000 glm2/24h. The term MTVR stands for "Moisture Vapour Transmission Rate", i.e. the amount of vapour which can pass per unit area during a certain period of time. Measurement of breathability has been performed with the method ASTM E96 and the measurement equipment Lyssy has been used.

The liquid-impermeable and breathable layer 3 can, for example, consist of a perforated polyethylene film, where the size of the holes has been chosen so that air and vapour may pass, but not liquid molecules. If an absorbent article with a more textile-like appearance is desired, a liquid-impermeable film in combination with an outer fibre layer is suitable.

Breathable and vapour-permeable but liquid-impermeable material for use in absorbent articles, such as nappies, incontinence products and sanitary towels, are previously known in different embodiments. Breathable materials can, as mentioned above, consist of perforated plastic films. One example of such films is described in US 5,628,737. The liquid-impermeable and breathable outer layer 3 can also consist of micro-porous plastic films, as is described in, for example, EP-A-0238200. Another example of breathable materials are so-called non-woven which are air-permeable and vapour-permeable and which, if required, have been treated with means to improve the liquid-impermeability, as is described in EP-A-0196654.

The liquid-impermeable outer layer 3 is breathable and vapour-permeable, as has been mentioned above. The elastic first piece is also breathable and vapour-permeable. As this is not required to be liquid-impermeable it may be arranged with a higher degree of breathability than the liquid-impermeable outer layer.

The inner layer 4 can be made from a liquid-permeable non-woven.

The second piece 2 is elongate and is arranged with its longitudinal direction at right angles in relation to the longitudinal direction of the first piece 1. The second piece 2 is connected by one end portion 6 to one longitudinal edge portion 7 of the first piece 1, centrally thereon. The connection can be made by means of, for example, adhesive, thermal bonding or ultrasonic bonding.

In Fig. 1, the arrows A and B illustrate how the first and second pieces are folded to form the absorbent article of pants-like shape shown in Figs. 2 and 3. The elastic piece 1 is folded in according to the arrows A shown to form the rear portion 8 and side portions 9, 10 of the article, while the second piece is folded upwards according to the arrows B to form the front portion 11 and crotch portion 12 of the article.

As can be seen most clearly from Fig. 3, one end portion 13 of the first piece is arranged so as to be overlapped a little by a first side edge portion 14 of the second piece, and the other end portion 15 of the first piece is in a corresponding manner arranged so as to be overlapped a little by a second side edge portion 16 of the second piece. These overlapping portions are interconnected by means of, for example, adhesive, thermal bonding or ultrasonic bonding.

In Fig. 4, the thickness of the second piece has been exaggerated for the sake of clarity. The figure shows an outer layer 3 consisting of a laminate of a plastic film 17 and a non-woven layer 18 arranged outside. The inner layer 4 can consist of a liquid-permeable non-woven of a type well-known to the person skilled in the art.

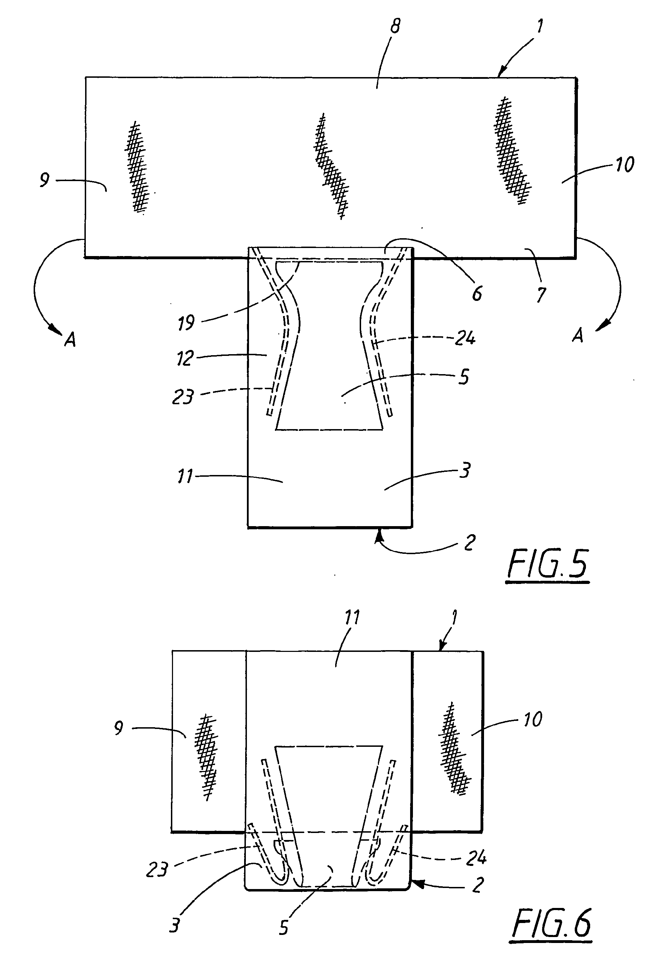

In the embodiment shown in Figs. 5 and 6, those parts corresponding to the same parts in the embodiment according to Figs. 1-4 have been provided with the same reference numbers. The only difference between the embodiment according to Figs. 5 and 6 and that described above is the shape and extent of the absorbent element 5. In this second embodiment, the absorbent element 5 is hourglass-shaped in plane form, as can be seen most clearly from Fig. 5, the narrower portion being intended to be arranged in the crotch of the wearer during use of the article. The upper end edge 19 of the absorbent element 5 is located directly adjacent to the lower edge portion 7 of the first elastic piece, which means that the absorbent element according to this second embodiment extends over the entire crotch area and a little way up over the front portion 11, as can be seen from Fig. 6. Elastic members 23,24 are arcuately arranged along the side edges of the absorbent element 5 and extend, as is evident from Fig. 5, from the upper elastic first piece 1 and down over the crotch portion of the article. As is evident from Fig. 5, the end portions of the elastic members 23,24 bridge that end portion 6 of the second piece which overlaps the first elastic piece 1. Thus, the elastic members are operatively connected, in the rear portion 8 of the article, with the first elastic piece 1. The opposite end of each elastic member 23,24 does not extend all the way to the side edges of the second piece 2, but are arranged so that the elastic members in the state of use of the articles, which is shown in Fig. 6, do not reach all the way to the side portions 9,10 of the first elastic piece. The relationship is the same in the embodiments according to Figs. 2 and 3.

Suitably, the elastic piece 1 and the elastic members have an elastic characteristic which allows an extension of up to the order of magnitude of twice the length with constant extension force. An absorbent article of a certain size can thereby be used on wearers of different sizes without the pressure from the elastic being varied, which in its turn leads to a very limited assortment of different sizes of the absorbent article being required in order to cover the need of sizes for wearers of different sizes.

In the embodiment shown in Figs. 5 and 6, the second piece 2 has been shaped rectangular. The second piece 2 may, of course, be provided with leg cut-outs which follow the curved elastic members and the curved edge portions of the hourglass-shaped absorbent element.

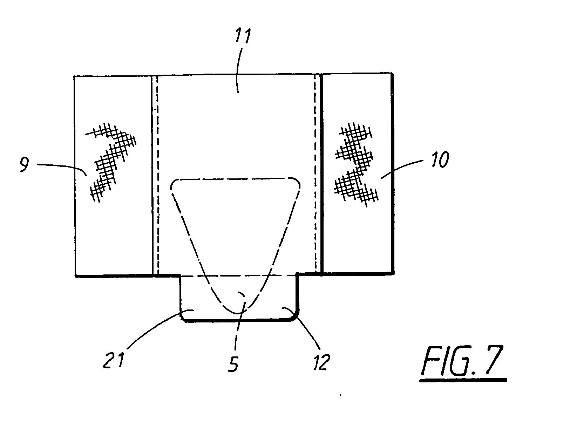

Fig. 7 shows a third embodiment which is slightly modified in relation to the first embodiment. The only difference is that a part piece 21 of the second piece is made narrower than the remainder of the second piece, in which way a pants-like article with a narrower crotch portion is obtained, as can be seen from Fig. 7.

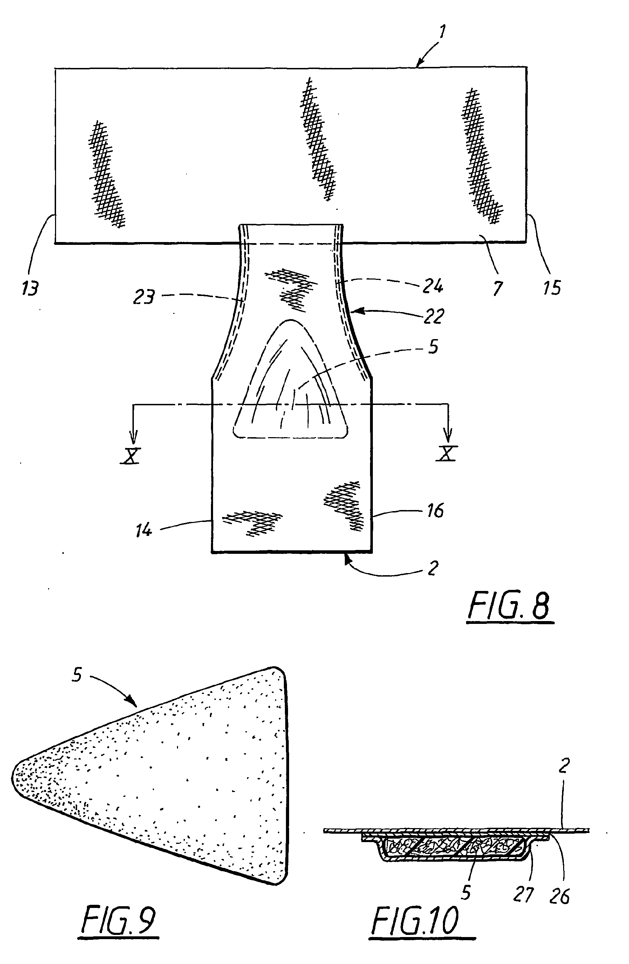

In the embodiment shown in Fig. 8, the parts corresponding to similar details in the other embodiments have been provided with the same reference numbers. The second piece 2 has a concave portion 22 which extends from the first piece 1 towards the side edge portions 14,16. These are, as in the above described embodiments, intended to be connected with the end portions 13 and 15 on the first piece 1.

The first piece 1 is formed of an elastic material an is intended during use of the article to achieve an elastic tightening around the waist of the wearer, which tightening keeps the article in place and gives it a good fit In the embodiment shown in Fig. 8, the elastic members 23,24 are arranged along both sides of the concave portion 22 for the forming of leg elastic, which during use of the article is intended to be in sealing contact with the legs of the wearer. In the shown embodiment, the elastic members 23, 24 extend from the elastic first piece 1 to the side edge portions 14,16. The complected, assembled, pants-like article will thereby be elastically tight-fitting around both of the legs of the wearer. In this manner a good fit is obtained. If the elastic piece 1 and the elastic members 23,24 are formed so that they are elastically stretchable a longer distance, without the tension in the elastic piece 1 and in the elastic members 23,24 being increased to any considerable extent, the article can, without any annoying chafing, be used by wearers of different sizes. In such a design, the number of requisite sizes can be kept down, which leads to savings and rational manufacturing compared to if there is a need for a larger assortment of sizes.

Fig. 9 schematically shows the absorbent element 5 according to a suitable embodiment. In order to avoid giving rise to annoying chafing in the crotch of the wearer, the absorbent element 5 has been designed as narrowing in that direction which during use of the article is directed rearwards. A complication with this narrowing design is that the absorbent element is narrowest in that portion which during use of the article is situated substantially in front of the genitals of the wearer, in which portion the demand for absorption capacity is greatest. To avoid the risk of leakage in the narrower portion of the absorbent element to the most practicable degree, the superabsorbent material, according to a suitably embodiment where the superabsorbent material is part of the absorbent element in the form of particles or fibres, has been arranged with an increasing concentration in said narrowing direction. This has been illustrated In Fig. 9 with the number of shown dots in correspondence with a gradient of the number of superabsorbent particles per unit volume of the absorbent element 5 in said direction.

In Fig. 4 an embodiment is shown where the liquid-impermeable but breathable outer layer 3 consists of a layer of the second piece. According to one embodiment, the liquid-impermeable outer layer is arranged only over the absorbent element, and forms an outer layer of said element In Fig. 10 a cross section along the line X-X in Fig. 8 is shown, in order to illustrate this. The absorbent element is here enclosed in a liquid-permeable layer 26, for example of non-woven, and of a liquid-impermeable and at the same time breathable layer 27, for example of a perforated plastic film. The two layers 26,27 extend beyond the absorbent element and a distance around it and are there interconnected. The unit formed by the absorbent element 5 and the two layers 26 and 27 is attached to the second piece 2 of the absorbent article, wherein the liquid-permeable layer 26 has been connected to the second piece 2, as is shown in Fig. 10.

The invention is not limited to the illustrative embodiments described above, but a number of modifications are possible within the scope of the patent claims below.

The absorption unit shown in Fig. 10, consisting of the absorbent element 5 and the layers 26 and 27, may be replaced with an absorbent foam material in one piece, which foam material is provided with an integral liquid-impermeable and at the same time air-permeable layer.

In Fig. 10 a separate liquid-impermeable layer 27 of the absorbent element is shown. Within the scope of the invention is also an embodiment where the absorbent element is made up of fibres, possibly with an admixture of SAP, and where the liquid-impermeable and breathable outer layer consists of an outer layer of hydrophobic fibres connected with the absorbent element.

Within the scope of the present invention are absorbent articles, in which different portions of the article through choices of different materials or degree of perforations, have different degrees of breathability.

When the expression liquid-impermeable and breathable has been used in the above text, it also implies that the material in question also is vapour-permeable, but that it does not allow passage of liquid drops.

In the above illustrative embodiments as is evident from Fig. 3, the side edge portions 14,16 of the second piece have been arranged on the outside of the end edge portions 13,15 of the first piece. It is, of course, possible to do the reverse, i.e. with the end edge portions 13,15 of the first piece farthest out. These two alternatives are to be preferred, even though it is within the scope of the invention to obtain said connection by means of attaching the inside of each of the end edge portions of the first piece to the inside of each side edge portion of the second piece, or by means of connecting corresponding edge portions outside to outside.

The article according to the invention has turned out to be especially suitable as incontinent shields for men. Other applications, such as menstrual shields or incontinence shields for women, are also conceivable.

In the above described illustrative embodiments, the highest absorption capacity per unit area for the narrowest area has been achieved by means of varying the content of superabsorbent material. Another method of achieving higher absorption capacity in the narrowest area is to form the absorbent element with a higher basis weight in this area. This can, for example, be carried out on an absorbent element formed from a fluff pulp of cellulose. Thus, the absorption capacity for an absorbent element of a fluff pulp may also be controlled by varying the density and/or the thickness in different portions.

The absorbent element can, of course within the scope of the invention, be formed from other materials, for example absorbent foam materials.

## Claims

1. Absorbent article in form of non-refastenable absorbent pants, such as incontinence pants, nappy pants or sanitary towel pants, said absorbent article has a longitudinal direction and a transverse direction and comprising an elastic waist portion, an absorbent element (5), which has a length and a width and is intended to at least cover the genitals of the wearer during use of the article, and a liquid-impermeable outer layer (3) which is intended to enclose the absorbent element on at least that side thereof which faces away from the wearer during use of the article,
**characterized in,**
**that** said elastic waist portion is made from an elastic first piece (1) which is elongate in a length direction parallel to said transverse direction and is intended to partly surround the trunk of the wearer and to form a rear portion (8) and side portions (9, 10) of the pants-like article,
in that a second piece (2) forming part of the article is designed to form a front portion (11) and a crotch portion (12) of the pants-like article,
in that said second piece (2) has a length and a width and is elongate with two opposite end edges and two opposite longitudinal side edges, in that the width of the second piece is, at least in the crotch portion (12), smaller than the length of the first piece (1),
in that the second piece (2) is arranged with its length direction at right angles in relation to the length direction of the first piece and is connected by a first end portion (6) to one edge portion (7) along the length direction of the first piece, centrally thereon,
in that one end portion (13) of the first piece is connected to a first side edge portion (14) of one of said side edges of the second piece, and in that the other end portion (15) of the first piece is connected in a corresponding manner to a second side edge portion (16) of the other of said side edges of the second piece, in addition to which the absorbent element (5) is in its entirety arranged on the second piece (2),
in that the liquid-impermeable outer layer (3), at least over a portion of this which covers the absorbent element, is breathable.

2. Absorbent article according to claim 1, **characterized in, that** the length of the second piece (2) is greater than the width of the first piece (1), and **in that** the projecting portion of the second piece formed by the length difference is in its entirety located below that side edge of the first piece which is the lower one during use of the article, and there forms the crotch portion (12).

3. Absorbent article according to claim 2, **characterized in, that** said projecting portion has a smaller width than the remainder of the second piece (2) (Fig. 7).

4. Absorbent element according to claim 2 or 3,
**characterized in,**
**that** the absorbent element (5) extends in its length direction over the entire crotch portion (12) and a little way up over the front portion (11) in the direction of that side edge of the first piece (1) which is the upper one during use of the article.

5. Absorbent article according to claim 2 or 3,
**characterized in,**
**that** the absorbent element (5) is arranged so as to extend with its lower end portion only a little way over the crotch portion (12) and over less than half the extent of said projecting portion (Fig. 2).

6. Absorbent article according to claim 5, **characterized in, that** the absorbent element (5) tapers in the direction of the crotch portion (12).

7. Absorbent article according to claim 4, **characterized in, that** the absorbent element (5) forms a concave shape with its side edge portions in the crotch area (Fig. 5).

8. Absorbent article according to any one of the preceding claims,
**characterized in,**
**that** said connected side edge portions (13, 15) and end portions (14, 16) of said first and second pieces (1 and, respectively, 2) are arranged in an overlapping manner, with the inside of an overlapping portion being arranged against the outside of an overlapped portion.

9. Absorbent article according to any one of the preceding claims,
**characterized in,**
**that** the second piece (2) includes a liquid-permeable inner layer (4) and said liquid-impermeable outer layer (3), and in that the absorbent element (5) is arranged between said inner and outer layers, the inner and outer layers extending in the lateral direction and longitudinal direction outside the absorbent element (5) and being interconnected there.

10. Absorbent article according to any one of claims 1-8,
**characterized in,**
**that** the liquid-impermeable and breathable outer layer (3) only covers the absorbent element.

11. Absorbent article according to any one of claim 10,
**characterized in,**
**that** the absorbent element is made up of fibres and that the liquid-impermeable and breathable outer layer consists of an outer layer of hydrophobic fibres connected with the absorbent element.

12. Absorbent article according to any one of the preceding claims,
**characterized in,**
**that** said breathable outer layer (3) has an MTVR-value between in the order of magnitude of 200-10000 g/m2/24h.

13. Absorbent article according to any one of the preceding claims,
**characterized in,**
**that** said breathable outer layer (3) has an MTVR-value in the order of magnitude of 500-5000 g/m2/24h.

14. Absorbent article according to any one of the preceding claims,
**characterized in,**
**that** said breathable outer layer (3) has an MTVR-value between in the order of magnitude of 1000-3000 g/m2/24h.

15. Absorbent article according to any one of the preceding claims,
**characterized in,**
**that** the entire second piece (2) is breathable and vapour-permeable.

16. Absorbent article according to any one of the preceding claims,
**characterized in,**
**that** the first piece (1) is rectangular.

17. Absorbent article according to any one of the preceding claims,
**characterized in,**
**that** the second piece (2) has a concave portion (22) between the first piece (1) and up to said side edge portions (14,16) of the second piece (2), wherein the concave portion forms cut-outs for the legs of the wearer.

18. Absorbent article according to any one of the preceding claims,
**characterized in,**
**that** the second piece (2) is, at least along a part of its length, narrowing towards the first piece (1), wherein the narrowing portion forms cut-outs for the legs of the wearer.

19. Absorbent article according to any one of the preceding claims,
**characterized in,**
**that** the front portion(1) is essentially inelastic.

20. Absorbent article according to any one of the preceding claims,
**characterized in,**
**that** elastic members (23,24) are arranged along the edges of the second piece (2), along at least the crotch portion for the forming of leg elastics, intended to lie in sealing contact with the legs of the wearer.

21. Absorbent article according to claim 20,
**characterized in, that** the elastic members (23,24) extend from an area (6) in the rear portion where the elastic first piece (1) and the second piece (2) overlap each other and further along the crotch portion along the edges of the second piece in a direction towards the front portion, whereby the elastic members are effectively connected with the elastic first piece.

22. Absorbent article according to any one of the preceding claims,
**characterized in, that** the liquid-impermeable outer layer (3) is breathable and vapour-permeable and that the rest of the article, over its entire area, is breathable and vapour-permeable.

23. Absorbent article according to claim 22,
**characterized in, that** different portions of the article due to a choice of different material or degree of perforation have different degrees of breathability.

24. Absorbent article according to claim 23,
**characterized in, that** the elastic first piece (1) has a higher degree of breathability than the liquid-impermeable outer layer on the second piece (2).

25. Absorbent article according to any one of the preceding claims,
**characterized in,**
**that** the absorbent element (5) comprises superabsorbent material and is continuously narrowing in a direction towards the rear portion of the article and that the concentration of superabsorbent material increases in the same direction.

26. Method of manufacturing an absorbent article in form of non-refasteable absorbent pants, such as incontinence pants, nappy pants or sanitary towel pants, said absorbent article has a longitudinal direction and a transverse direction and comprising an elastic waist portion, an absorbent element (5), which has a length and a width and is intended to at least cover the genitals of the wearer during use of the article, and a liquid-impermeable outer layer (3) which is intended to enclose the absorbent element on at least that side thereof which faces away from the wearer during use of the article,
**characterized in,**
**that** said elastic waist portion is made from an elastic first piece (1) which is essentially rectangular with a length and a width and is intended in use to partly surround the trunk of the wearer and to form a rear portion (8) and side portions (9, 10) of the pants-like article, in that a second piece (2) forming part of the article is designed to form a front portion (11) and a crotch portion (12) of the pants-like article,
in that said second piece (2) is made with a length and a width and is arranged with its length direction along the longitudinal direction with two opposite end edges and two opposite longitudinal side edges, in that the width of the second piece (2) is designed, at least in the crotch portion (12), smaller than the length of the first piece (1), in that the second piece is arranged with its length direction at right angles in relation to the length direction of the first piece and is connected by a first end portion (6) to one edge portion (7) along the length direction of the first piece, centrally thereon, in that one end portion (13) of the first piece (1) is connected to a first side edge portion (14) of one of said side edges of the second piece (2), and in that the other end portion (15) of the first piece (1) is connected in a corresponding manner to a second side edge portion (16) of the other of said side edges of the second piece, and that at least a portion of the liquid-impermeable outer layer, which portion is intended to cover the absorbent element, is formed from a breathable material.

27. Method according to claim 26, **characterized in,**
**that** the absorbent element (5) in its entirety is arranged on the second piece (2) forming part of the article before said piece is connected to said first piece (1).

28. Method according to claim 27, **characterized in,**
**that** the absorbent element (5), before being arranged on the second piece (2), is provided with a liquid-impermeable and breathable layer (27).

## Patentansprüche

1. Absorbierender Artikel in Form einer nicht-wiederbefestigbaren absorbierenden Unterhose, wie z.B. einer Inkontinenzunterhose, einer Windelhose oder einer Bindeunterhose, wobei der absorbierende Artikel eine Längsrichtung und eine Querrichtung aufweist und einen elastischen Taillenteil, ein absorbierendes Element (5), das eine Länge und eine Breite aufweist und dazu vorgesehen ist, mindestens die Genitalien des Benutzers während der Anwendung des Artikels zu decken, und eine flüssigkeitsundurchlässige Außenschicht (3), welche dazu vorgesehen ist, das absorbierende Element auf mindestens derjenigen Seite davon einzuschließen, die während der Anwendung des Artikels dem Benutzer abgewandt ist, umfasst,
**dadurch gekennzeichnet,**
**dass** der elastische Taillenteil aus einem elastischen ersten Stück (1) hergestellt ist, welches in einer zur Querrichtung parallelen Längsrichtung länglich ist und dazu vorgesehen ist, den Torso des Benutzers teilweise zu umgeben und einen Hinterteil (8) und Seitenteile (9, 10) des unterhosenähnlichen Artikels zu bilden,
**dass** ein zweites Stück (2), welches einen Teil des Artikels bildet, zur Bildung eines Vorderteils (11) und eines Schrittteils (12) des unterhosenähnlichen Artikels ausgebildet ist,
**dass** das zweite Stück (2) eine Länge und eine Breite aufweist und mit zwei gegenüberliegenden Endkanten und zwei gegenüberliegenden Längsseitenkanten länglich ist, dass die Breite des zweiten Stücks, zumindest in dem Schrittteil (12), kleiner als die Länge des ersten Stücks (1) ist,
**dass** das zweite Stück (2) mit seiner Längsrichtung rechtwinklig zur Längsrichtung des ersten Stücks angeordnet ist und durch einen ersten Endteil (6) mit einem Kantteil (7) entlang der Länge des ersten Stücks, mittig darauf, verbunden ist,
**dass** ein Endteil (13) des ersten Stücks mit einem ersten Seitenkantteil (14) einer der Seitenkanten des zweiten Stücks verbunden ist, und dass der andere Endteil (15) des ersten Stücks auf entsprechende Art und Weise mit einem zweiten Seitenkantteil (16) der anderen der Seitenkanten des zweiten Stücks verbunden ist, wobei ferner das absorbierende Element (5) in seiner Gesamtheit auf dem zweiten Stück (2) angeordnet ist,
**dass** die flüssigkeitsundurchlässige Außenschicht (3), zumindest in einem Bereich von dieser, der das absorbierende Element deckt, atmungsaktiv ist.

2. Absorbierender Artikel nach Anspruch 1, **dadurch gekennzeichnet, dass** die Länge des zweiten Stücks (2) größer als die Breite des ersten Stücks (1) ist, und dass der vorstehende Teil des zweiten Stücks, der durch den Längenunterschied gebildet ist, in seiner Gesamtheit unter derjenigen Seitenkante des ersten Stücks angeordnet ist, die während der Anwendung des Artikels die untere ist, und dort den Schrittteil (12) bildet.

3. Absorbierender Artikel nach Anspruch 2, **dadurch gekennzeichnet, dass** der vorstehende Teil eine kleinere Breite als der übrige Teil des zweiten Stücks (2) (Fig. 7) aufweist.

4. Absorbierendes Element nach Anspruch 2 oder 3, **dadurch gekennzeichnet, dass** sich das absorbierende Element (5) in seiner Längsrichtung über den ganzen Schrittteil (12) und um eine kleine Strecke über den Vorderteil (11) hinauf in Richtung derjenigen Seitenkante des ersten Stücks (1), die während der Anwendung des Artikels die obere ist, erstreckt.

5. Absorbierender Artikel nach Anspruch 2 oder 3, **dadurch gekennzeichnet, dass** das absorbierende Element (5) derart angeordnet ist, dass es sich mit seinem unteren Endteil nur um eine kleine Strecke über den Schrittteil (12) hinauf und über weniger als die Hälfte der Erstreckung des vorstehenden Teils (Fig. 2) erstreckt.

6. Absorbierender Artikel nach Anspruch 5, **dadurch gekennzeichnet, dass** sich das absorbierende Element (5) in Richtung des Schrittteils (12) verjüngt.

7. Absorbierender Artikel nach Anspruch 4, **dadurch gekennzeichnet, dass** das absorbierende Element (5) mit seinen Seitenkantteilen im Schrittbereich (Fig. 5) eine konkave Form bildet.

8. Absorbierender Artikel nach einem der vorgehenden Ansprüche, **dadurch gekennzeichnet, dass** die verbundenen Seitenkantteile (13, 15) und Endteile (14,16) des ersten und zweiten Stücks (1 bzw. 2) überlappend angeordnet sind, wobei die Innenseite eines überlappenden Teils gegen die Außenseite eines überlappten Teils angeordnet ist.

9. Absorbierender Artikel nach einem der vorgehenden Ansprüche, **dadurch gekennzeichnet, dass** das zweite Stück (2) eine flüssigkeitsdurchlässige Innenschicht (4) und die flüssigkeitsundurchlässige Außenschicht (3) umfasst, und dass das absorbierende Element (5) zwischen der Innen- und der Außenschicht angeordnet ist, wobei sich die Innen- und die Außenschicht in der Seitenrichtung und Längsrichtung außerhalb des absorbierenden Elements (5) erstrecken und dort miteinander verbunden sind.

10. Absorbierender Artikel nach einem der Ansprüche 1-8, **dadurch gekennzeichnet, dass** die flüssigkeitsundurchlässige und atmungsaktive Außenschicht (3) nur das absorbierende Element deckt.

11. Absorbierender Artikel nach Anspruch 10, **dadurch gekennzeichnet, dass** das absorbierende Element aus Fasern gebildet ist, und dass die flüssigkeitsundurchlässige und atmungsaktive Außenschicht aus einer außenseitigen Schicht von hydrophoben Fasern besteht, welche mit dem absorbierenden Element verbunden sind.

12. Absorbierender Artikel nach einem der vorgehenden Ansprüche, **dadurch gekennzeichnet, dass** die atmungsaktive Außenschicht (3) einen MTVR-Wert in einer Größenordnung von 200-10000 g/m2/24h aufweist.

13. Absorbierender Artikel nach einem der vorgehenden Ansprüche, **dadurch gekennzeichnet, dass** die atmungsaktive Außenschicht (3) einen MTVR-Wert in einer Größenordnung von 500-5000 g/m2/24h aufweist.

14. Absorbierender Artikel nach einem der vorgehenden Ansprüche, **dadurch gekennzeichnet, dass** die atmungsaktive Außenschicht (3) einen MTVR-Wert in einer Größenordnung von 1000-3000 g/m2/24h aufweist.

15. Absorbierender Artikel nach einem der vorgehenden Ansprüche, **dadurch gekennzeichnet, dass** der gesamte zweite Stück (2) atmungsaktiv und dampfdurchlässig ist.

16. Absorbierender Artikel nach einem der vorgehenden Ansprüche, **dadurch gekennzeichnet, dass** das erste Stück (1) rechteckig ist.

17. Absorbierender Artikel nach einem der vorgehenden Ansprüche, **dadurch gekennzeichnet, dass** das zweite Stück (2) zwischen dem ersten Stück (1) bis zu den Seitenkantteilen (14, 16) des zweiten Stücks (2) einen konkaven Teil (22) aufweist, wobei der konkave Teil Ausschnitte für die Beine des Benutzers bildet.

18. Absorbierender Artikel nach einem der vorgehenden Ansprüche, **dadurch gekennzeichnet, dass** das zweite Stück (2) zumindest entlang einem Teil seiner Länge gegen das erste Stück (1) schmaler wird, wobei der eingeengte Teil Ausschnitte für die Beine des Benutzers bildet.

19. Absorbierender Artikel nach einem der vorgehenden Ansprüche, **dadurch gekennzeichnet, dass** der Vorderteil (1) im Wesentlichen unelastisch ist.

20. Absorbierender Artikel nach einem der vorgehenden Ansprüche, **dadurch gekennzeichnet, dass** elastische Elemente (23, 24) entlang den Kanten des zweiten Stücks (2), entlang mindestens dem Schrittteil für die Bildung von Beingummibändern angeordnet sind, die zur abdichtenden Anlage an den Beinen des Benutzers vorgesehen sind.

21. Absorbierender Artikel nach Anspruch 20, **dadurch gekennzeichnet, dass** sich die elastischen Elemente (23, 24) von einem Bereich (6) im Hinterteil ausgehend, wo das elastische erste (1) und zweite Stück (2) sich überlappen, und weiter entlang dem Schrittteil entlang den Kanten des zweiten Stücks in eine Richtung gegen den Vorderteil erstrecken, wobei die elastischen Elemente mit dem elastischen ersten Stück wirksam verbunden sind.

22. Absorbierender Artikel nach einem der vorgehenden Ansprüche, **dadurch gekennzeichnet, dass** die flüssigkeitsundurchlässige Außenschicht (3) atmungsaktiv und dampfdurchlässig ist, und dass der übrige Teil des Artikels über seine gesamte Fläche atmungsaktiv und dampfdurchlässig ist.

23. Absorbierender Artikel nach Anspruch 22, **dadurch gekennzeichnet, dass** verschiedene Teile des Artikels wegen der Wahl eines unterschiedlichen Materials oder der Wahl eines unterschiedlichen Perforierungsgrads verschiedene Atmungsaktivitäten aufweisen.

24. Absorbierender Artikel nach Anspruch 23, **dadurch gekennzeichnet, dass** das elastische erste Stück (1) eine höhere Atmungsaktivität als die flüssigkeitsundurchlässige Außenschicht des zweiten Stücks (2) aufweist.

25. Absorbierender Artikel nach einem der vorgehenden Ansprüche, **dadurch gekennzeichnet, dass** das absorbierende Element (5) superabsorbierendes Material aufweist und sich in eine Richtung auf den Hinterteil des Artikels zu kontinuierlich verjüngt, und dass sich die Konzentration superabsorbierenden Materials in dieselbe Richtung erhöht.

26. Verfahren zur Herstellung eines absorbierenden Artikels in Form einer nicht-wiederbefestigbaren absorbierenden Unterhose, wie z.B. einer Inkontinenzunterhose, einer Windelhose oder einer Bindeunterhose, wobei der absorbierende Artikel eine Längsrichtung und eine Querrichtung aufweist und einen elastischen Taillenteil, ein absorbierendes Element (5), das eine Länge und eine Breite aufweist und dazu vorgesehen ist, mindestens die Genitalien des Benutzers während der Anwendung des Artikels zu decken, und eine flüssigkeitsundurchlässige Außenschicht (3), die dazu vorgesehen ist, das absorbierende Element auf mindestens derjenigen Seite davon einzuschließen, die während der Anwendung des Artikels dem Benutzer abgewandt ist, umfasst,
**dadurch gekennzeichnet,**
**dass** der elastische Taillenteil aus einem elastischen ersten Stück (1) hergestellt ist, das im Wesentlichen rechteckig ist und eine Länge und eine Breite aufweist und dazu vorgesehen ist, während der Anwendung den Torso des Benutzers teilweise zu umgeben und einen Hinterteil (8) und Seitenteile (9, 10) des unterhosenähnlichen Artikels zu bilden, dass ein einen Teil des Artikels bildendes zweites Stück (2) dazu ausgebildet ist, einen Vorderteil (11) und einen Schrittteil (12) des unterhosenähnlichen Artikels zu bilden,
**dass** das zweite Stück (2) mit einer Länge und einer Breite gebildet ist und mit seiner Längenrichtung entlang der Längsrichtung mit zwei gegenüberliegenden Endkanten und zwei gegenüberliegenden Längsseitenkanten angeordnet ist, dass die Breite des zweiten Stücks (2) zumindest im Schrittteil (12) kleiner als die Länge des ersten Stücks (1) gebildet ist, dass das zweite Stück mit seiner Längsrichtung rechtwinklig zur Längsrichtung des ersten Stücks angeordnet ist und durch einen ersten Endteil (6) mit einem Kantteil (7) entlang der Längsrichtung des ersten Stücks, mittig darauf, verbunden ist, dass ein Endteil (13) des ersten Stücks (1) mit einem ersten Seitenkantteil (14) einer der Seitenkanten des zweiten Stücks (2) verbunden ist, und dass der andere Endteil (15) des ersten Stücks (1) auf entsprechende Art und Weise mit einem zweiten Seitenkantteil (16) der anderen der Seitenkanten des zweiten Stücks verbunden ist, und dass zumindest ein Teil der flüssigkeitsundurchlässigen Außenschicht, welcher Teil zum Decken des absorbierenden Elements vorgesehen ist, aus einem atmungsaktiven Material gebildet ist.

27. Verfahren nach Anspruch 26, **dadurch gekennzeichnet, dass** das absorbierende Element (5) in seiner Gesamtheit auf dem zweiten Stück (2) angeordnet ist, wodurch es vor dem Verbinden mit dem ersten Stück (1) einen Teil des Artikels bildet.

28. Verfahren nach Anspruch 27, **dadurch gekennzeichnet**, das absorbierende Element (5) vor dem Anbringen auf dem zweiten Stück (2) mit einer flüssigkeitsundurchlässigen und atmungsaktiven Schicht (27) versehen ist.

## Revendications

1. Article absorbant dans la forme de culotte absorbante et non rattachable, comme une culotte d'incontinence, des couches pour bébés ou des pantalons de serviettes hygiéniques, ledit article absorbant présentant une direction longitudinale et une direction transversale et comprenant une portion de ceinture élastique, un élément absorbant (5) qui présente une longueur et une largeur et est destiné à recouvrir au moins les parties génitales de l'utilisateur lors de l'utilisation de l'article, et une couche extérieure imperméable aux liquides (3) qui est destinée à enfermer l'élément absorbant sur au moins ce côté de celui-ci qui est détourné de l'utilisateur pendant l'utilisation de l'article,
**caractérisé en**
**ce que** ladite portion de ceinture élastique est faite à partir d'une première pièce élastique (1) qui est allongée dans une direction longitudinale parallèle à ladite direction transversale et est destinée à entourer partiellement le tronc de l'utilisateur et à former une partie arrière (8) et des parties latérales (9, 10) de l'article en forme de culotte,
**en ce qu'**une deuxième pièce (2) faisant partie de l'article est conçue pour former une partie avant (11) et une partie d'entre-jambes (12) de l'article en forme de culotte,
**en ce que** ladite deuxième pièce (2) présente une longueur et une largeur et est allongée avec deux bords d'extrémité opposés et deux bords latéraux longitudinaux opposés, **en ce que** la largeur de la deuxième pièce est, au moins dans la partie d'entre-jambes (12), inférieure à la longueur de la première pièce (1),
**en ce que** la deuxième pièce (2) est agencée avec sa direction longitudinale perpendiculaire à la direction de longueur de la première pièce et est reliée par une première portion d'extrémité (6) à l'une portion de bord (7) le long de la direction de longueur de la première pièce, au milieu de celle-ci,
**en ce que** l'une portion d'extrémité (13) de la première pièce est reliée à une première partie de bord latérale (14) de l'un desdits bords latéraux de la deuxième pièce, et ce que l'autre partie d'extrémité (15) de la première pièce est reliée d'une manière correspondante à une deuxième partie de bord latérale (16) de l'autre desdits bords latéraux de la deuxième pièce, et en outre l'élément absorbant (5) est disposé dans sa totalité sur la deuxième pièce (2),
**en ce que** la couche extérieure imperméable aux liquides (3), au moins sur une partie de celle-ci recouvre l'élément absorbant, est perméable à l'air.

2. Article absorbant selon la revendication 1, **caractérisé en ce que** la longueur de la deuxième pièce (2) est supérieure à la largeur de la première pièce (1), et **en ce que** la partie en saillie de la deuxième pièce formée par la différence de longueur est dans sa totalité située au-dessous de ce bord latéral de la première pièce qui est le plus bas lors de l'utilisation de l'article, et y forme la partie d'entre-jambes (12).

3. Article absorbant selon la revendication 2, **caractérisé en ce que** ladite partie en saillie présente une largeur inférieure au reste de la deuxième pièce (2) (Fig. 7).

4. Elément absorbant selon la revendication 2 ou 3, **caractérisé en ce que** l'élément absorbant (5) s'étend dans sa direction longitudinale sur toute la partie d'entre-jambes (12) et un peu plus haut sur la partie avant (11) dans la direction de ce bord latéral de la première pièce (1) qui est le plus haut lors de l'utilisation de l'article.

5. Article absorbant selon la revendication 2 ou 3, **caractérisé en ce que** l'élément absorbant (5) est agencé de manière à s'étendre avec sa partie d'extrémité inférieure seulement un peu au-dessus de la portion d'entrejambes (12) et au-dessus de moins que la moitié de l'étendue de ladite partie en saillie (Fig. 2).

6. Article absorbant selon la revendication 5, **caractérisé en ce que** l'élément absorbant (5) se rétrécit dans la direction de la portion d'entre-jambes (12).

7. Article absorbant selon la revendication 4, **caractérisé en ce que** l'élément absorbant (5) crée une forme concave avec ses parties de bord latérales dans la zone d'entre-jambes (Fig. 5).

8. Article absorbant selon l'une quelconque des revendications précédentes, **caractérisé en ce que** lesdites parties de bord latérales (13, 15) et parties d'extrémité (14, 16) reliées desdites première et deuxième pièces (1 et, respectivement, 2) sont arrangées d'une manière chevauchante, avec l'intérieur d'une partie chevauchante étant arrangée contre l'extérieur d'une partie chevauchée.

9. Article absorbant selon l'une quelconque des revendications précédentes, **caractérisé en ce que** la deuxième pièce (2) comprend une couche intérieure perméable aux liquides (4) et ladite couche extérieure imperméable aux liquides (3), et **en ce que** l'élément absorbant (5) est disposé entre lesdites couches intérieure et extérieure, les couches intérieure et extérieure s'étendant dans la direction latérale et la direction longitudinale à l'extérieur de l'élément absorbant (5) et y étant reliés entre elles.

10. Article absorbant selon l'une quelconque des revendications 1 à 8, **caractérisé en ce que** la couche extérieure imperméable aux liquides et perméable à l'air (3) ne couvre que l'élément absorbant.

11. Article absorbant selon la revendication 10, **caractérisé en ce que** l'élément absorbant est constitué de fibres, et que la couche extérieure imperméable aux liquides et perméable à l'air se compose d'une couche extérieure de fibres hydrophobes reliée à l'élément absorbant.

12. Article absorbant selon l'une quelconque des revendications précédentes, **caractérisé en ce que** ladite couche extérieure perméable à l'air (3) présente une valeur de MTVR comprise dans l'ordre de grandeur de 200 à 10000 g/m2/24h.

13. Article absorbant selon l'une quelconque des revendications précédentes, **caractérisé en ce que** ladite couche extérieure perméable à l'air (3) présente une valeur de MTVR comprise dans l'ordre de grandeur de 500 à 5000 g/m2/24h.

14. Article absorbant selon l'une quelconque des revendications précédentes, **caractérisé en ce que** ladite couche extérieure perméable à l'air (3) présente une valeur de MTVR comprise dans l'ordre de grandeur de 1000 à 3000 g/m2/24h.

15. Article absorbant selon l'une quelconque des revendications précédentes, **caractérisé en ce que** la totalité de la deuxième pièce (2) est perméable à l'air et perméable à la vapeur.

16. Article absorbant selon l'une quelconque des revendications précédentes, **caractérisé en ce que** la première pièce (1) est rectangulaire.

17. Article absorbant selon l'une quelconque des revendications précédentes, **caractérisé en ce que** la deuxième pièce (2) comporte une partie concave (22) entre la première pièce (1) et vers le haut par rapport auxdites parties de bord latérales (14, 16) de la deuxième pièce (2), la partie concave formant des découpes pour les jambes de l'utilisateur.

18. Article absorbant selon l'une quelconque des revendications précédentes, **caractérisé en ce que** la deuxième pièce (2) est, au moins le long d'une partie de sa longueur, rétrécissante vers la première pièce (1), la partie rétrécissante formant des découpes pour les jambes de l'utilisateur.

19. Article absorbant selon l'une quelconque des revendications précédentes, caractérisé en en que la partie avant (1) est essentiellement non élastique.

20. Article absorbant selon l'une quelconque des revendications précédentes, **caractérisé en ce que** les éléments élastiques (23,24) sont disposés le long des bords de la deuxième pièce (2), le long d'au moins la partie d'entre-jambes pour la formation des élastiques de jambe, destinés à se trouver en contact d'étanchéité avec les jambes de l'utilisateur.

21. Article absorbant selon la revendication 20, **caractérisé en ce que** les éléments élastiques (23, 24) s'étendent à partir d'une zone (6) dans la partie arrière, où la première pièce élastique (1) et la deuxième pièce (2) se chevauchent mutuellement et en outre le long de la partie d'entre-jambes le long des bords de la deuxième pièce dans une direction vers la partie avant, les éléments élastiques étant reliés de manière efficace avec le premier élément élastique.

22. Article absorbant selon l'une quelconque des revendications précédentes, **caractérisé en ce que** la couche extérieure imperméable aux liquides (3) est perméable à l'air et perméable à la vapeur et que le reste de l'article, sur toute sa surface, est perméable à l'air et perméable à la vapeur.

23. Article absorbant selon la revendication 22, **caractérisé en ce que** les différentes parties de l'article en raison d'un choix de matériaux différents ou de taux de perforation présentent différents degrés de respirabilité.

24. Article absorbant selon la revendication 23, **caractérisée en ce que** la première pièce élastique (1) présente un degré de respirabilité supérieur à celui de la couche extérieure imperméable aux liquides sur la deuxième pièce (2).

25. Article absorbant selon l'une quelconque des revendications précédentes, caractérisé en que l'élément absorbant (5) comprend un matériau superabsorbant et ne cesse de se rétrécir dans une direction vers la partie arrière de l'article et que la concentration de matériau superabsorbant augmente dans la même direction.

26. Procédé de fabrication d'un article absorbant dans la forme de culotte absorbante et non rattachable, comme une culotte d'incontinence, des couches pour bébés ou des pantalons de serviettes hygiéniques, ledit article absorbant présentant une direction longitudinale et une direction transversale et comprenant une portion de ceinture élastique, un élément absorbant (5) qui présente une longueur et une largeur et est destiné à recouvrir au moins les parties génitales de l'utilisateur lors de l'utilisation de l'article, et une couche extérieure imperméable aux liquides (3) qui est destinée à enfermer l'élément absorbant sur au moins ce côté de celui-ci qui est détourné de l'utilisateur pendant l'utilisation de l'article,
**caractérisé en**
**ce que** ladite portion de ceinture élastique est faite à partir d'une première pièce élastique (1) qui est essentiellement rectangulaire avec une longueur et une largeur et est destinée à entourer partiellement le tronc de l'utilisateur et à former une partie arrière (8) et des parties latérales (9, 10) de l'article en forme de culotte, **en ce qu'**une deuxième pièce (2) faisant partie de l'article est conçue pour former une partie avant (11) et une partie d'entre-jambes (12) de l'article en forme de culotte,
**en ce que** ladite deuxième pièce (2) est faite avec une longueur et une largeur et est arrangée avec sa direction de longueur le long de la direction longitudinale avec deux bords d'extrémité opposés et deux bords latéraux longitudinaux opposés, **en ce que** la largeur de la deuxième pièce (2) est conçue, au moins dans la partie d'entre-jambes (12), comme étant inférieure à la longueur de la première pièce (1), **en ce que** la deuxième pièce est agencée avec sa direction de longueur perpendiculaire à la direction de longueur de la première pièce et est reliée par une première portion d'extrémité (6) à l'une portion de bord (7) le long de la direction de longueur de la première pièce, au milieu de celle-ci, **en ce que** l'une portion d'extrémité (13) de la première pièce (1) est reliée à une première partie de bord latérale (14) de l'un desdits bords latéraux de la deuxième pièce (2), et ce que l'autre partie d'extrémité (15) de la première pièce (1) est reliée d'une manière correspondante à une deuxième partie de bord latéral (16) de l'autre desdits bords latéraux de la deuxième pièce, et ce qu'au moins une portion de la couche extérieure imperméable aux liquides, portion qui est destinée à recouvrir l'élément absorbant, est formée à partir d'un matériau perméable à l'air.

27. Procédé selon la revendication 26, **caractérisé en ce que** l'élément absorbant (5) dans son ensemble est disposé sur la deuxième pièce (2) faisant partie de l'article avant que ladite pièce ne soit reliée à ladite première pièce (1).

28. Procédé selon la revendication 27, **caractérisé en ce que** l'élément absorbant (5), avant d'être disposé sur la deuxième pièce (2), est muni d'une couche imperméable aux liquides et perméable à l'air (27).
